(19) Europäisches Patentamt
European Patent Office
Office européen des brevets

(11) Numéro de publication: **0 031 753**
**B1**

(12) **FASCICULE DE BREVET EUROPEEN**

(45) Date de publication du fascicule du brevet: **19.09.84**

(21) Numéro de dépôt: **80401788.7**

(22) Date de dépôt: **12.12.80**

(51) Int. Cl.³: **C 07 D 401/06,**
C 07 D 401/14,
C 07 D 409/14, A 61 K 31/47
// C07D211/34

(54) **Nouveaux dérivés de la (pipéridyl-4)-2 (quinolyl-4)-1 éthanone, produits intermédiaires et procédés pour leur préparation, et leur utilisation comme médicaments.**

(30) Priorité: **21.12.79 FR 7931396**

(43) Date de publication de la demande:
**08.07.81 Bulletin 81/27**

(45) Mention de la délivrance du brevet:
**19.09.84 Bulletin 84/38**

(84) Etats contractants désignés:
**BE CH DE FR GB IT LI LU NL SE**

(56) Documents cités:
**FR-A-2 177 511**
**FR-A-2 354 771**

**E. SCHRÖDER et al. Arzneimittelchemie II, Georg, Thilme Verlag Stuttgart, page 34 (1976)**

(73) Titulaire: **PHARMUKA LABORATOIRES**
**35 Quai du Moulin de Cage**
**F-92231 Gennevilliers (FR)**

(72) Inventeur: **Champseix, Alain André**
**15, rue Clémenceau**
**F-91406 Orsay (FR)**
Inventeur: **Le Fur, Gérard Roger**
**35, rue du Progrès**
**F-92350 Plessis-Robinson (FR)**
Inventeur: **Renault, Christian Louis Albert**
**17 bis, Allée Réaumur**
**F-95150 Taverny (FR)**

(74) Mandataire: **Gaumont, Robert et al**
**RHONE-POULENC RECHERCHES Service Brevets Pharma 25, Quai Paul Doumer**
**F-92408 Courbevoie Cedex (FR)**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 031 753 B1**

## Description

La présente invention a pour objet de nouveaux dérivés de la (pipéridyl-4)-2 (quinolyl-4)-1 éthanone utilisables comme médicaments ou comme produits intermédiaires pour la fabrication de médicaments.

Il est déjà connu par le brevet français FR—A—2 354 771 des dérivés de la [(quinolyl)-4)-3 propyl-1]-4 pipéridine ayant une activité psychotrope et antidépressive.

Les dérivés selon l'invention peuvent être représentés par la formule générale:

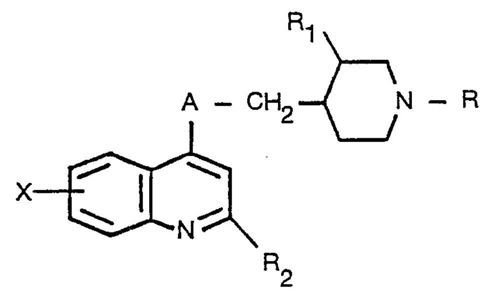

(I)

dans laquelle R représente un atome d'hydrogène, un groupe alcényle ayant 3 ou 4 atomes de carbone, un groupe alkyle comportant de 1 à 4 atomes de carbone ou un groupe phénylalkyle, dont la partie alkyle comporte 1 ou 2 atomes de carbone, $R_1$ représente un atome d'hydrogène, un groupe alkyle ayant 1 à 4 atomes de carbone ou un groupe alcényle ayant 2 à 4 atomes de carbone, $R_2$ représente un groupe phényle, pyridyle ou thiényle, ou un groupe phényle substitué par un ou deux substituants pris parmi les atomes d'halogène (chlore, fluor, brome, iode) et les groupes alkyle ou alcoxy ayant 1 à 4 atomes de carbone, X représente un atome d'hydrogène ou un atome d'halogène (chlore, fluor, brome, iode) fixé en position 6 sur le cycle de la quinoléine.

A représente un groupe CO ou un groupe $CH_2$.

Dans la formule (I) ci-dessus, $R_2$ est de préférence un groupe phényle ou phényle substitué.

Lorsque $R_1$ n'est pas l'atome d'hydrogène, la molécule des composés de formule (I) comporte deux atomes de carbone asymétriques et donc, pour une signification donnée de R, $R_1$, $R_2$, A et X il y a deux diastéréoisomères, dénommés respectivement composé cis et composé trans selon que le groupe $R_1$ et le groupe fixé en position 4 sur le cycle pipéridinique sont en position cis ou en position trans l'un par rapport à l'autre. Ces deux diastéréoisomères peuvent être représentés schématiquement par les formules ($I_a$) et ($I_b$) suivantes:

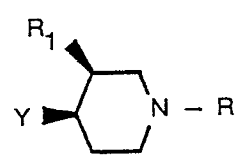

($I_a$) Composé cis

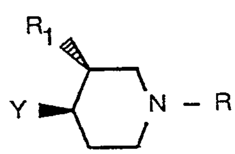

($I_b$) Composé trans

formules dans lesquelles Y représente le groupe:

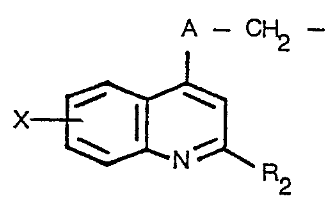

A chaque diastéréoisomère correspond un racémique et deux énantiomères, chaque énantiomère correspondant à une configuration absolue déterminée des carbones en positions 3 et 4 du cycle pipéridinique.

Les divers isomères signalés ci-dessus font partie de l'invention, de même que les sels d'addition des composés répondant à la formule globale (I) avec les acides minéraux ou organiques.

Les composés de formule générale (I) pour lesquels A représente le groupe $CH_2$ peuvent être préparés par réduction des produits correspondants de formule générale (I) pour lesquels A représente le groupe CO, selon le schéma réactionnel suivant:

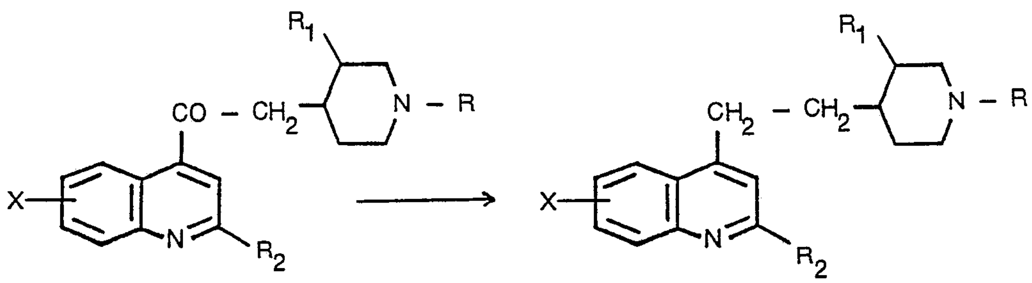

Pour cette réduction on utilise des méthodes, connues en soi, qui permettent de transformer un groupement CO en groupement $CH_2$, par exemple celles décrites par R. B. WAGNER et H. D. ZOOK (Synthetic Organic Chemistry, p.5, J. WILEY and SONS — 1953). On utilise avantageusement comme agent réducteur l'hydrate d'hydrazine en présence d'un hydroxyde de métal alcalin tel que l'hydroxyde de sodium ou de potassium, au sein d'un solvant inerte tel qu'un alcool ou un diol, par exemple le diéthylèneglycol, à une température comprise entre 100 et 180°C.

Les produits de formule générale (I) pour lesquels A représente le groupe CO et R l'atome d'hydrogène peuvent être préparés par condensation d'un ester d'acide quinoléine carboxylique-4 de formule (II) avec un ester d'acide pipéridine-4 acétique de formule (III) puis hydrolyse et décarboxylation du composé de formule (IV) ainsi obtenu, selon le schéma réactionnel suivent:

a)

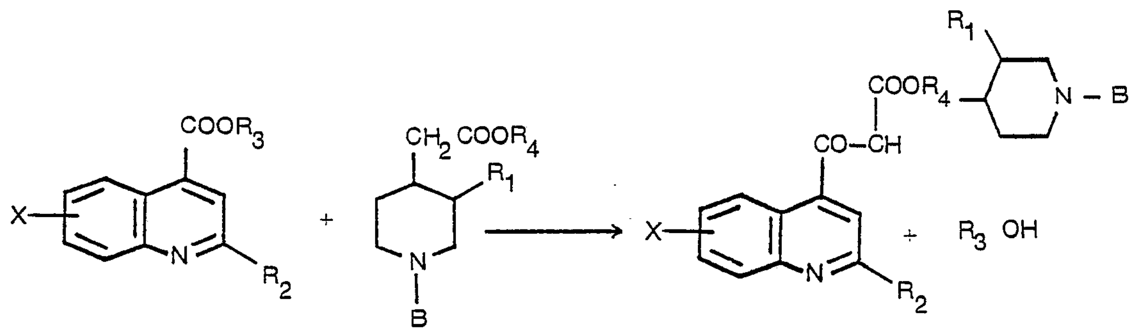

(II) (III) (IV)

b)

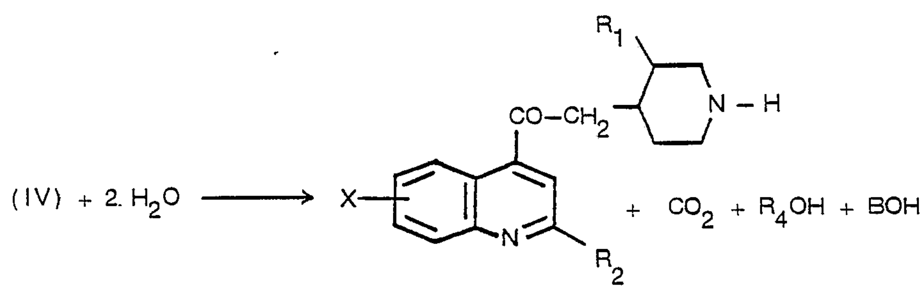

Dans les formules ci-dessus $R_3$ et $R_4$ représentent des groupes alkyle de bas poids moléculaire, par exemple méthyle ou éthyle, et B représente un groupe protecteur de la fonction amine, stable en milieu alcalin anhydre et susceptible d'être éliminé en milieu acide, tels que ceux qui sont décrit par R. A. BOISSONNAS, Advances in Organic Chemistry *3*, p. 159, Interscience (1963). On utilise avantageusement le groupe benzoyle ($—B=—CO—C_6H_5$) ou le groupe benzyloxycarbonyle ($—B=—CO—O—CH_2—C_6H_5$).

Pour réaliser la réaction de condensation a) on fait appel à des procédés connus en soi (cf. "The acetoacetic acid ester condensation", C. R. HAUSER et al., Organic Reactions, vol.1, p.266, WILEY and SONS, 1942). On opère avantageusement en présence d'une base telle qu'un alcoolate (par exemple le tertiobutylate de potassium) ou un hydrure métallique (par exemple l'hydrure de sodium ou de potassium), au sein d'un solvant inerte tel qu'un hydrocarbure ou un autre solvent aprotique (par exemple le tétrahydrofuranne), à une température comprise entre 0°C et la température d'ébullition du solvant utilisé.

La réaction d'hydrolyse b) est conduite selon des procédés connus en soi (cf. "Cleavage of β-ketoesters", R. B. WAGNER et H. D. ZOOK, Synthetic Organic Chemistry, p.327, WILEY and SONS, 1953). La méthode la plus courante consiste à chauffer à l'ébullition le produit de formule (IV) dans une solution aqueuse d'un acide tel que l'acide chlorhydrique ou l'acide sulfurique.

Les produits de départ de formule (II) sont facilement accessibles par des méthodes décrites dans la littérature (cf. Quinolines — Heterocyclic compounds — *32*, 274, WILEY and SONS, 1977). Il en est de même pour les composé de formule (III) (cf. W. E. DOERING et J. D. CHANCERY J. Am. Chem. Soc. 1946, *68*, 586; V. PRELOG. Helv. Chim. Acta. 1944, *27*, 535; R. F. BORNE J. Heterocycl. Chem. 1972, *9*, 869).

Les composés de formule générale (I) pour lesquels R est un groupe alkyle, alcényle ou phénylalkyle peuvent être préparés par action sur les composés correspondants de formule (I) pour lesquels R est un atome d'hydrogène d'un agent alkylant.

Comme agents alkylant on peut citer en particulier les halogénures de formule R Hal, les sulfates de formule $(R)_2SO_4$ et les alkyl- ou arylsulfonates de formule $ArSO_3R$ ou $R'SO_3R$, formules dans lesquelles R représente un groupe alkyle ayant 1 à 4 atomes de carbone, un groupe alcényle ayant 3 ou 4 atomes de carbone ou un groupe phénylalkyle dont la partie alkyle a 1 ou 2 atomes de carbone, Ar représente un groupe aryle et R' représente un groupe alkyle. La réaction peut être schématisée comme suit dans le cas où on utilise comme agent alkylant un halogénure:

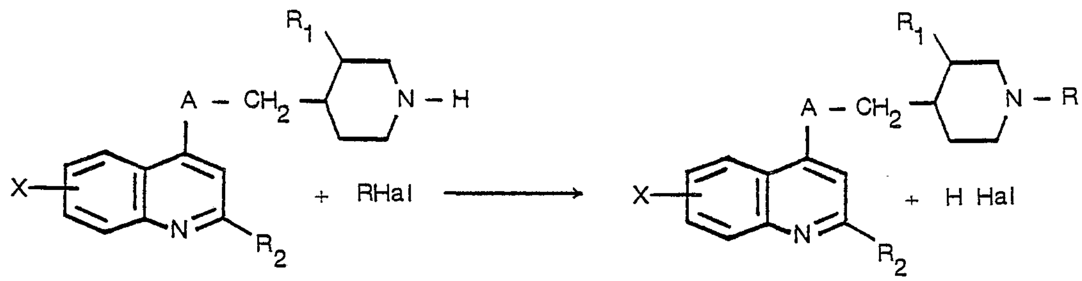

La réaction d'alkylation des composés de formule (I) pour lesquels R = H au moyen de l'agent alkylant est effectuée selon des procédés connus en soi, tels que ceux décrits par R. B. WAGNER et H. D. ZOOK (Synthetic Organic Chemistry, p.666, J. WILEY and SONS, 1965). On opère avantageusement en présence d'une base organique ou minérale (par exemple le carbonate de sodium ou de potassium), au sein d'un solvant inerte, par exemple le diméthylformamide.

Une variante par la préparation des produits de formule générale (I) pour lesquels A représente le groupe $CH_2$ et R un groupe alkyle ou phénylalkyle consiste à faire réagir sur les produits de formule (I) correspondants pour lesquels A représente le groupe $CH_2$ et R l'atome d'hydrogène un chlorure d'acide ou un ester chloroformique de formule

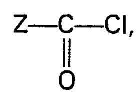

dans laquelle Z représente un groupe alkyle ayant 1 à 3 atomes de carbone, un groupe phényle, un groupe phénylalkyle dont la partie alkyle a un atome de carbone ou un groupe alcoxy bas (par exemple méthoxy ou éthoxy), et à réduire ensuite au moyen d'un hydrure le composé de formule (V) ainsi obtenu, selon le schéma réactionnel suivant:

c)

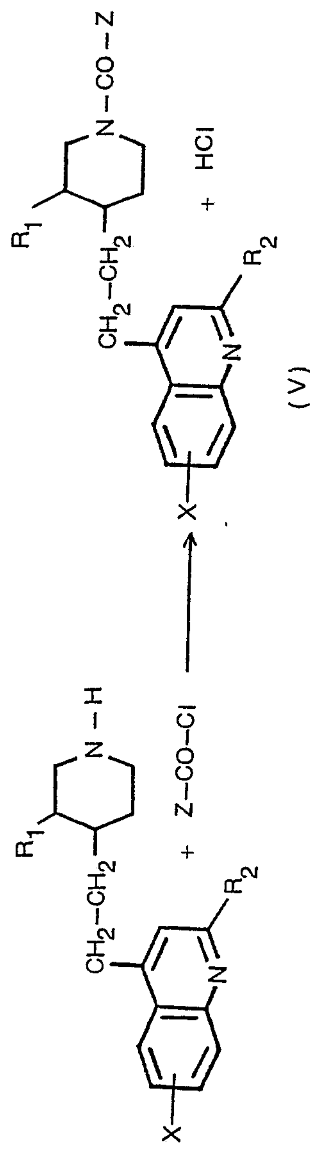

(V)

d) (V)

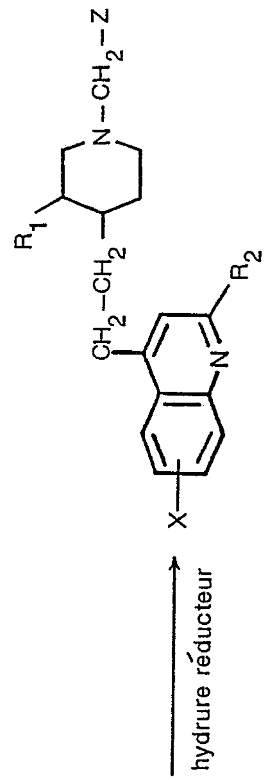


d) bis (V)

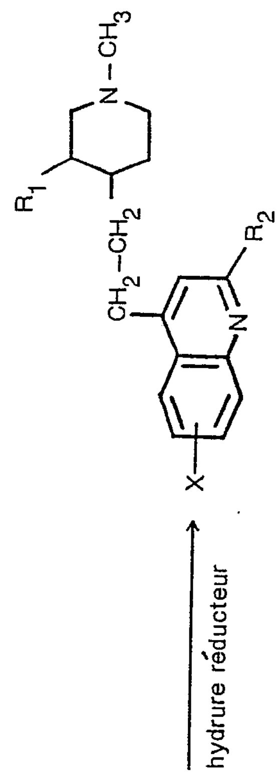

La réaction d) correspond au cas où Z est alkyle, phényl ou phénylalkyle, la réaction d) bis au cas où Z est alcoxy bas.

Pour effecteur la réaction c) on utilise des méthodes, connues en soi, qui permettent de transformer une amine secondaire en amide (cas Z = alkyle, phényle ou phénylalkyle) ou en carbamate (cas Z = alcoxy), par exemple celles décrites par R. B. WAGNER et H. D. ZOOK (Synthetic Organic Chemistry, p.565 et p.646, J. WILEY and SONS, 1953). On opère généralement en présence d'une base telle que l'hydroxyde de sodium en solution aqueuse ou le triéthylamine, au sein d'un solvant inerte tel que le chloroforme ou le trichloro-1,1,1 éthane, à une température comprise entre 0°C et 30°C.

Les réactions d) et d) bis utilisent également des méthodes connues (cf. par exemple R. B. WAGNER et H. D. ZOOK, Synthetic Organic Chemistry, p. 660, J. WILEY and SONS, 1953). On utilise avantageusement comme hydrure réducteur l'hydrure d'aluminium et de lithium ou d'autres hydrures complexes tels que l'hydrure de sodium et de bis (méthoxy-2 éthoxy) aluminium, au sein d'un solvant inerte tel qu'un éther ou un hydrocarbure aromatique.

Les produits de formule (I) pour lesquels A représente le groupe $CH_2$, R l'atome d'hydrogène peuvent être préparés également à partir des produits de formule (I bis) selon la schéma réactionnel suivant en quatre étapes:

e) 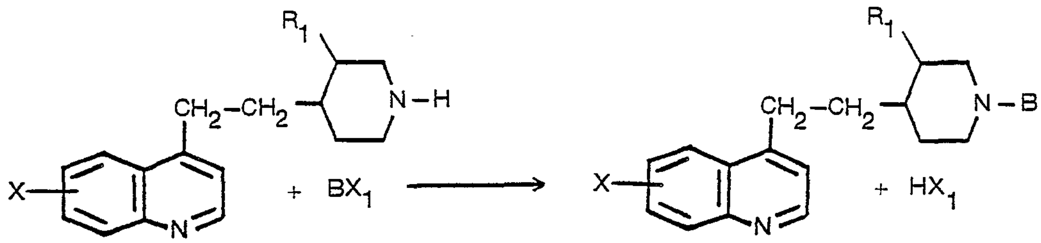

(I bis) (VI)

f) 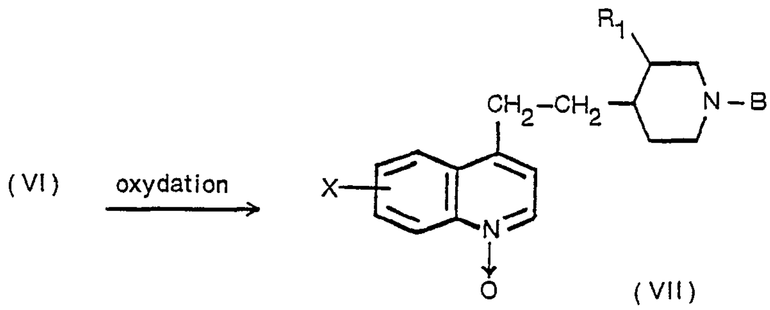


(VII)

g) 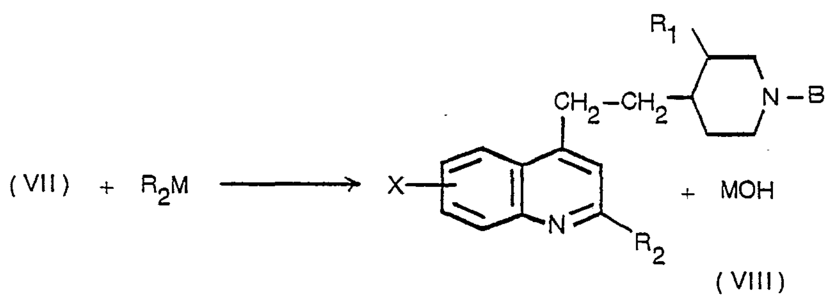

(VIII)

h) 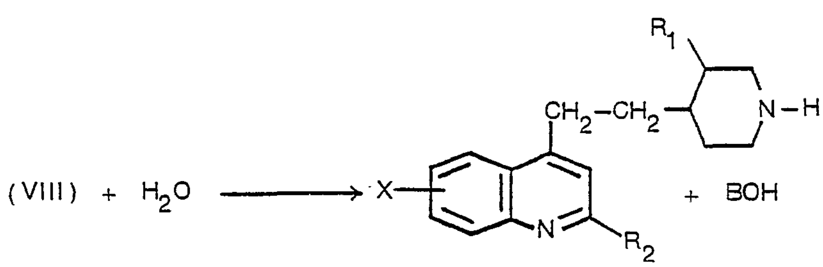

La réaction e), qui a pour but de protéger la fonction amine pipéridinique, consiste à faire réagir un composé de formule (I bis) avec un composé $BX_1$ permettant le remplacement de l'atome d'hydrogène fixé sur l'azote du cycle pipéridinique par un groupe protecteur B, stable en présence de composés organométalliques et susceptible d'être éliminé en milieu acide. Les groupes protecteurs généralement utilisés sont des groupes acyle décrits par R. A. BOISSONNAS (Advances in Organic Chemistry, 3, page 159, Interscience, 1963), par exemple le groupe benzoyle. Comme composé $BX_1$ permettant le remplacement ci-dessus on peut citer les halogénures d'acyle, en particulier le chlorure de benzoyle.

La réaction e) est alors réalisée dans des conditions analogues à celles utilisées pour la réaction c).

Pour réaliser la réaction d'oxydation f) on utilise les méthodes, connues en soi, qui permettent d'oxyder sur l'azote les hétérocycles azotés (cf. par exemple A. R. KATRITZKY et Coll., Chemistry of the Heterocyclic N-oxydes, Organic Chemistry, *19*, p.21, Academic Press, 1971). On utilise avantageusement comme agent oxydant des péroxydes tels que les acides monoperphtalique, paranitroperbenzoïque ou métachloroperbenzoïque, au sein d'un solvant inerte tel que l'éther, à une température comprise entre 0°C et 25°C.

La réaction g) consiste à faire réagir sur le N-oxyde obtenu dans l'étape précédente un composé organométallique de formule $R_2M$, dans laquelle $R_2$ a les mêmes significations que dans la formule (I) et M représente un métal alcalin, en particulier le lithium, ou le radical MgHal, Hal désignant un atome d'halogène, dans les conditions décrites par A. R. KATRITZKY et Coll. (même référence que ci-dessus, p.308). On opère avantageusement en présence d'un excès de composé organométallique, au sein d'un solvant inerte tel que l'éther, le tétrahydrofuranne ou un hydrocarbure aromatique, à une température de 0°C à 25°C.

La réaction d'hydrolyse h) est réalisée dans des conditions analogues à celles utlisées pour la réaction b).

Les produits de formule générale (I) pour lesquels A représente le groupe $CH_2$, R l'atome d'hydrogène et $R_1$ l'atome d'hydrogène ou un groupe alkyle 1 à 4 C peuvent être préparés également par réaction du monochlorocarbène: CHCl sur les dérivés de l'indole de formule (IX), selon la schéma réactionnel suivant:

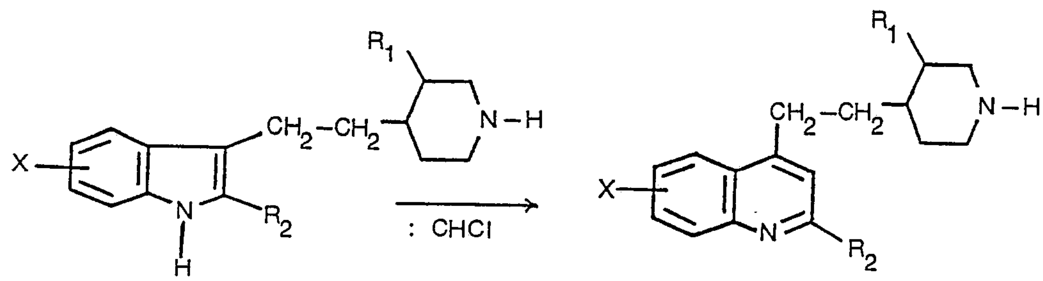


(IX)

Dans la formule (IX) ci-dessus $R_1$ est un atome d'hydrogène ou un groupe alkyle 1 à 4 C et X et $R_2$ ont les mêmes significations que dans la formule (I).

Le monochlorocarbène est préparé "in situ" par action d'une base sur le dichlorométhane, selon des méthodes connues (cf. par exemple H. DOBBS, Chem. Comm. *1965*, 56 et J. Org. Chem. 1968, *33*, 1093; CLOSS, J. Org. Chem. 1961, *26*, 2609 et Tetrah. Letters *1976*, 3179). On utilise avantageusement comme base le méthyllithium au sein d'un solvant inerte tel que l'éther ou le tétrahydrofuranne, à une température comprise entre −30°C et +20°C.

Les dérivés indoliques de formule (IX) peuvent être préparés selon des procédés connus (cf. par exemple brevet français 2 334 358, brevet anglais 1 023 781 et J. Org. Chem. 1961, 26, 3368—3371).

Les composés de formule générale (I) pour lesquels R est l'atome d'hydrogène et $R_1$ le groupe éthényle (vinyle) et pour lesquels l'atome de carbone du cycle pipéridinique porteur du groupe éthényle $R_1$ a une configuration donnée, rectus (R) ou sinister (S), peuvent être préparés par chauffage à température supérieure à 50°C, au sein d'un solvant protique ou d'un mélange de solvants protiques, en présence ou en l'absence de formaldéhyde, des composés de formule (I) correspondants pour lesquels R est l'atome d'hydrogène et $R_1$ le groupe éthényle et pour lesquels l'atome de carbone du cycle pipéridinique porteur du groupe éthényle $R_1$ a la configuration inverse, sinister (S) ou rectus (R), partiellement ou totalement salifiés. Le chauffage peut être réalisé en particulier dans un milieu aqueux acide de pH voisin de 6, à une température de 120 à 160°C. Ce chauffage entraîne une isomérisation des composés de départ.

Les mélanges réactionnels obtenus par les divers procédés décrits précédemment sont traités suivant des méthodes classiques, physiques (évaporation, extraction à l'aide d'un solvant, distillation, cristallisation, chromatographie, etc...) ou chimiques (formation de sel et régénération de la base, etc...) afin d'isoler les composés de formule (I) à l'état pur.

Les composés de formule (I) sous forme de base libre peuvent éventuellement être transformés en sels d'addition avec un acide minéral ou organique par action d'un tel acide au sein d'un solvant approprié.

Les médicaments de la classe des benzodiazépines sont utilisés comme anticonvulsivants, comme hypnotiques et pour le traitement des états d'anxiété et de divers états psychonévrotiques. La présence de récepteurs spécifiques des benzodiazépines dans les membranes de cerveau de rat a été démontrée [SQUIRES et Coll., Nature, *266*, (1977), 732] et le degré d'affinité des benzodiazépines pour ces récepteurs, degré d'affinité mesuré par leur aptitude à déplacer de ses sites de liaison le Diazépam tritié, est en bonne corrélation avec les effets pharmacodynamiques observés chez l'animal et chez l'homme.

Jusqu'à ce jour, en dehors des benzodiazépines, aucun médicament agissant par ailleurs sur le système nerveux central ne s'est montré capable de déplacer, de manière significative, le Diazépam de ses sites de liaison [cf BRAESTRUP et Coll., Europ. J. Pharmacol. 48, (1978) 26].

Les produits de l'invention, bien que de structure différente de celles des benzodiazépines, déplacement le Diazépam de ses sites de liaison. Ils peuvent donc trouver des applications comme hypnotiques, comme anticonvulsivants, et dans le traitement des états de tension et d'anxiété résultant de circonstances "stressantes" ou de troubles somatiques liés à des facteurs émotionnels. Ils sont utilisables pour le traitement des états psychonévrotiques se manifestant par des symptômes d'anxiété, d'appréhension, de fatigue, d'agitation ou de dépression.

Les produits de l'invention possèdent d'autre part des propriétés antiarythmiques.

Les exemples suivants illustrent l'invention sans la limiter. Les données relatives aux spectres de résonance magnétique nucléaire (en abrégé: spectres R.M.N.) figurant dans ces exemples concernent la résonance magnétique nucléaire des protons des composés à l'état de base. Pour effectuer les mesures de résonance magnétique nucléaire les composés sont mis en solution dans le chloroforme deutéré.

### Exemple 1

(Phenyl-2 quinolyl-4)-1 (piperidyl-4)-2 ethanone

A une suspension de 27,5 g de t-butylate de potassium dans 215 ml de tétrahydrofuranne sec, placée sous atmosphère d'azote et refroidie à 0°C, on ajoute rapidement une solution de 21,2 g de phényl-2 quinoléinecarboxylate-4 de méthyle dans 50 ml de tétrahydrofuranne sec. La température étant maintenue inférieure à +10°C, on introduit lentement en 2 heures une solution de 22,1 g de (benzoyl-1 pipéridyl-4) acétate d'éthyle dans 80 ml de tétrahydrofuranne sec. Le mélange réactionnel est ensuite agité pendant 20 heures à température ambiante, puis on amène à sec par évaporation du solvant. Le résidu est chauffé au reflux pendant 18 heures dans 650 ml d'une solution aqueuse 5 N d'acide chlorhydrique.

Après refroidissement, la solution obtenue est filtrée et le filtrat extrait par 2 fois 250 ml d'éther. La solution aqueuse résiduelle est concentrée sous pression réduite. Le résidu obtenu est extrait par 500 ml de méthanol chaud et la solution d'extraction est filtrée. Le filtrat, après évaporation du méthanol, fournit 13,8 g de dichlorhydrate de (phényl-2 quinolyl-4)-1 (pipéridyl-4)-2 éthanone fondant à 259°C.

Spectre R.M.N. du produit obtenu:

aromatiques $\delta$: 7,3—8,2 ppm

—$CH_2N$ et $CH_2CO$ $\delta$: 2,2—3,2 ppm

### Exemple 2

[(Fluoro-4 phenyl)-2 Quinolyl-4]-1 (piperidyl-4)-2 ethanone

A 250 ml de tétrahydrofuranne sec, sous atmosphère d'azote, on ajoute lentement, par portions, 12 g d'hydrure de sodium sous forme d'une suspension à 50% dans l'huile de vaseline. La température étant maintenue vers 20°C, on introduit rapidement une solution de 15 g de (benzoyl-1 pipéridyl-4) acétate d'éthyle et de 16 g de (fluoro-4 phényl)-2 quinoléinecarboxylate-4 d'éthyle dans 250 ml de tétrahydrofuranne sec. La suspension obtenue est laissée 15 heures à température ambiante, puis chauffée pendant 6 heures au reflux. Le mélange réactionnel est évaporé à sec, le résidu est repris par 100 ml de chlorure de méthylène, et on amène de nouveau à sec. Au résidu obtenu on ajoute très lentement 300 ml d'une solution aqueuse 11 N d'acide chlorhydrique et le mélange final est chauffé pendant 15 heures au reflux. La solution aqueuse obtenue est extraite par 2 fois 500 ml d'éther. La solution aqueuse résiduelle est alcalinisée au moyen d'une solution aqueuse 11 N d'hydroxyde de sodium, puis extraite par 2 fois 300 ml de chlorure de méthylène. Après évaporation du chlorure de méthylène, on obtient 17,8 g de [(fluoro-4 phényl)-2 quinolyl-4]-1 (pipéridyl-4)-2 éthanone, dont le fumarate, formé par action de l'acide fumarique au sein de l'éthanol, fond à 206°C.

Spectre R.M.N. du produit obtenu:

| | |
|---|---|
| aromatiques | $\delta$: 7—8,4 ppm |
| $CH_2N$ et $COCH_2$ | $\delta$: 2,4—3,4 ppm |
| $CH_2$—(CH) | $\delta$: 1—2,2 ppm |

Exemple 3

[(Chloro-4 phenyl)-2 quinolyl-4]-1 (piperidyl-4)-2 ethanone

On opère comme dans l'exemple 2, en partant de 22 g d'hydrure de sodium (suspension à 50% dans l'huile), 29,7 g de (chloro-4 phényl)-2 quinoléinecarboxylate-4 de méthyle et 26,1 g de (benzoyl-1 pipéridyl-4) acétate de méthyle. On obtient finalement 22,2 g de [(chloro-4 phényl)-2 quinolyl-4]-1 (pipéridyl-4)-2 éthanone dont le monométhanesulfonate, formé par action de l'acide méthanesulfonique au sein de l'éthanol, fond à 170°C.

Spectre R.M.N. du produit obtenu:

| | |
|---|---|
| aromatiques | $\delta$: 7,2—8,4 ppm |
| $CH_2N$ et $COCH_2$ | $\delta$: 2,3—3,4 ppm |
| $CH_2$—(CH) | $\delta$: 1—2,2 ppm |

Exemple 4

[(Methyl-4 phenyl)-2 quinolyl-4]-1 (piperidyl-4)-2 ethanone

A 16,8 g de t-butylate de potassium dans 100 ml de tétrahydrofuranne sec on ajoute 14,5 g de (méthyl-4 phényl)-2 quinoléinecarboxylate-4 d'éthyle puis, goutte-à-goutte, une solution de 13,7 g de (benzoyl-1 pipéridyl-4) acétate d'éthyle dans 150 ml de tétrahydrofuranne sec.

On agite 18 heures à la température ambiante, élimine le solvant par distillation sous pression réduite et reprend le résidu par 300 ml d'une solution aqueuse 5 N d'acide chlorhydrique. On porte au reflux 4 heures, lave la solution aqueuse par 2 fois 100 ml d'ether, l'alcalinise par addition d'hydroxyde de sodium et extrait par 3 fois 200 ml de chlorure de méthylène. On lave la solution organique à l'eau, la sèche et l'évapore sous pression réduite.

On obtient 15,2 g de [(méthyl-4-phényl)-2 quinolyl-4]-1 (pipéridyl-4)-2 éthanone, dont le dichlorhydrate fond au dessus de 260°C.

Spectre R.M.N. du produit obtenu:

| | |
|---|---|
| aromatiques | $\delta$: 7,2—8,4 ppm |
| $CH_2N$ et $CH_2CO$ | $\delta$: 2,2—3,2 ppm |
| $CH_3Ar$ | $\delta$: 2,4 ppm |

Exemple 5

[(Methoxy-4 phenyl)-2 Quinolyl-4]-1 (piperidyl-4)-2 ethanone

On opère comme dans l'exemple 4, en partant de 28,5 g de t-butylate de potassium, 25 g de (méthoxy-4 phényl)-2 quinoléinecarboxylate-4 de méthyle et 22,2 g de (benzoyl-1 pipéridyl-4) acétate de méthyle.

On obtient 21,6 g de [(méthoxy-4 phényl)-2 quinolyl-4]-1 (pipéridyl-4)-2 éthanone, dont le dichlorhydrate fond au dessus de 260°C.

Spectre R.M.N. du produit obtenu:

| | |
|---|---|
| aromatiques | $\delta$: 6,9—8,4 ppm |
| $CH_2N$ et $COCH_2$ | $\delta$: 2,4—3,4 ppm |
| $CH_3O$ | $\delta$: 3,8 ppm |

Exemple 6

[Ethenyl-3 (R) piperidyl-4 (S)]-2 (phenyl-2 quinolyl-4)-1 ethanone

On opère comme dans l'exemple 4, en partant de 39,9 g de t-butylate de potassium, 30,8 g de phényl-2 quinoléinecarboxylate-4 de méthyle et 35,4 g de [benzoyl-1 éthényl-3 (R) pipéridyl-4 (S)] acetate d'éthyle. On obtient finalement 8 g d'[éthényl-3 (R) pipéridyl-4 (S)]-2 (phényl-2 quinolyl-4)-1 éthanone sous forme de dichlorhydrate qui fond à 210°C.

Spectre R.M.N. du produit obtenu:

$COCH_2$ δ: 3 ppm

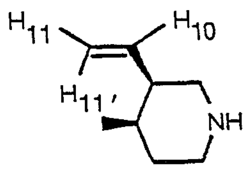

$\delta H_{10}$: 6,2 ppm

$\delta H_{11}'$: 5 ppm

Exemple 7

Phenyl-2 [(piperidyl-4)-2 ethyl]-4 quinoleine

On porte à 180°C pendant 15 minutes une solution de 63,3 g de (phényl-2 quinolyl-4)-1 (pipéridyl-4)-2 éthanone, préparée comme indiqué à l'exemple 1, et de 29,3 g d'hydrate d'hydrazine à 98% dans 300 ml de diéthylèneglycol. On refroidit à 60°C, introduit 32 g d'hydroxyde de potassium et porte à 180°C pendant deux heures. On dilue avec 500 ml d'eau et extrait avec 3 fois 200 ml de chlorure de méthylène. On lave la phase organique à l'eau, la sèche et l'évapore à sec sous pression réduite. On obtient une huile, que l'on soumet à l'action de l'acide chlorhydrique au sein de l'éthanol.

On obtient 53,6 g de phényl-2 [(pipéridyl-4)-2 éthyl]-4 quinoléine sous forme de monochlorhydrate fondant à 219°C.

Spectre R.M.N. du produit obtenu:

aromatiques δ: 7,3—8,3 ppm

$CH_2Ar$ et $CH_2$—N δ: 2,2—3,2 ppm

Exemple 8

(Chloro-4 phenyl)-2 [(piperidyl-4)-2 ethyl]-4 quinoleine

On opère comme à l'exemple 7, en partant de 6 g de [(chloro-4 phényl)-2 quinolyl-4]-1 (pipéridyl-4)-2 éthanone, préparée comme indiqué à l'exemple 3, 2,5 g d'hydrate d'hydrazine à 98% et 2,73 g d'hydroxyde de potassium dans 25 ml de diéthylène glycol. On obtient 2,1 g de (chloro-4 phényl)-2 [(pipéridyl-4)-2 éthyl]-4 quinoléine sous forme de monochlorhydrate, qui fond au-dessus de 260°C.

Spectre R.M.N. du produit obtenu:

aromatiques δ: 7,3—8,2 ppm

$CH_2Ar$ et N—$CH_2$ δ: 2,2—3,4 ppm

Exemple 9

(Methoxy-4 phenyl)-2 [(piperidyl-4)-2 ethyl]-4 quinoleine

A 10,8 g de [(méthoxy-4 phényl)-2 quinolyl-4]-1 (pipéridyl-4)-2 éthanone préparée comme indiqué à l'exemple 5, en solution dans 50 ml de diéthylène glycol, on ajoute 5,2 g d'hydrate d'hydrazine à 98% et on chauffe le milieu réactionnel pendant 30 minutes à 160°C. La température est ensuite ramenée à 120°C puis on introduit, en une fois, 5,6 g d'hydroxyde de potassium en pastilles. Le mélange est ensuite chauffé pendant 20 heures à 160°C, puis versé dans 450 ml d'eau. On extrait par 240 ml de chloroforme. La phase chloroformique est lavée par 250 ml d'eau, séchée sur sulfate de magnésium, puis concentrée. Le résidu est traité par une solution d'acide chlorhydrique dans l'éthanol. On obtient 7,1 g de (méthoxy-4 phényl)-2 [(pipéridyl-4)-2 éthyl]-4 quinoléine sous forme de monochlorhydrate fondant au-dessus de 260°C.

Spectre R.M.N. du produit obtenu:

aromatiques δ: 6,8—8,2 ppm

$CH_2N$ et $CH_2$—Ar δ: 2,1—3,3 ppm

$CH_3O$ δ: 3,8 ppm

Exemple 10

(Methyl-4 phenyl)-2 [(piperidyl-4)-2 ethyl]-4 quinoleine

On procède comme dans l'exemple 9, en partant de 20 g de [(méthyl-4 phényl)-2 quinolyl-4]-1 (pipéridyl-4)-2 éthanone, préparée comme indiqué à l'exemple 4, 5,6 g d'hydrate d'hydrazine et 2,4 g d'hydroxyde de sodium en solution dans 200 ml de diéthylène glycol. On obtient, après acidification du

résidu par une solution éthanolique d'acide chlorhydrique, 6,2 g de (méthyl-4 phényl)-2 [(pipéridyl-4)-2 éthyl]-4 quinoléine sous forme de monochlorhydrate fondant à 252°C.

Spectre R.M.N. du produit obtenu:

| | |
|---|---|
| aromatiques | δ: 7,1—8,2 ppm |
| $CH_3$—Ar | δ: 2,3 ppm |
| $CH_2$—N et $CH_2$—AR | δ: 2,2—3,3 ppm |

Exemple 11

[(Methyl-4 phenyl)-2 quinolyl-4]-1 [(phenyl-2 ethyl)-1 piperidyl-4]-2 ethanone

A une solution de 1,72 g de [(méthyl-4 phényl)-2 quinolyl-4]-1 (pipéridyl-4)-2 éthanone, préparée comme indiqué à l'exemple 4, dans 25 ml de diméthylformamide, on ajoute 3,45 g de carbonate de potassium et 1,39 g de bromure de phényl-2 éthyle. La suspension, bien agitée, est chauffée pendant 5 heures à 60°C, puis versée dans 1 litre d'eau glacée. L'huile qui relargue est décantée, lavée à l'eau, puis on ajoute 100 ml d'éther et 100 ml d'une solution aqueuse 1 N d'acide chlorhydrique. Le précipité formé est filté, lavé à l'eau puis à l'acétone. On obtient 2 g de [(méthyl-4 phényl)-2 quinolyl-4]-1 [(phényl-2 éthyl-1 pipéridyl-4]-2 éthanone sous forme de dichlorhydrate fondant à 195°C.

Spectre R.M.N. du produit obtenu:

| | |
|---|---|
| aromatiques | δ: 7—8,3 ppm |
| $CH_3Ar$ | δ: 2,4 ppm |
| $CH_2N$ et $CH_2CO$ | δ: 2,2—3,2 ppm |

Exemple 12

{[(Phenyl-2 ethyl)-1 piperidyl-4]-2 ethyl}-4 phenyl-2 quinoleine

On opère comme à l'exemple 11, en partant de 9,3 g de monochlorhydrate de phényl-2 [(pipéridyl-4)-2 éthyl]-4 quinoléine, préparé comme indiqué à l'exemple 7, 7,3 g de bromure de phenyl-2 éthyle et 18,1 g de carbonate de potassium, en suspension dans 130 ml de diméthylformamide. On obtient 8,3 g {[(phényl-2 éthyl)-1 pipéridyl-4]-2 éthyl}-4 phényl-2 quinoléine fondant à 80°C.

Spectre R.M.N. du produit obtenu:

| | |
|---|---|
| aromatiques | δ: 7,1—8,2 ppm |
| $CH_2N$ et $CH_2Ar$ | δ: 2,4—3,8 ppm |
| $C_6H_5$ | δ: 7,2 ppm |

Exemple 13

[(Methyl-1 piperidyl-4)-2 ethyl]-4 phenyl-2 quinoleine

5 g d'hydrure d'aluminium et de lithium sont introduits progressivement, par portions, dans 200 ml de tétrahydrofuranne sec, sous atmosphère d'azote. La suspension obtenue est refroidie à 0°C, puis on ajoute, goutte-à-goutte, und solution de 20 g d'[(éthoxycarbonyl-1 pipéridyl-4)-2 éthyl]-4 phényl-2 quinoléine dans 200 ml de tétrahydrofuranne, la température du mélange réactionnel étant maintenue au-dessous de 30°C. Après 4 heures de contact à la température ambiante, on introduit très lentement et successivement 5,85 ml d'eau, 4,3 ml d'une solution aqueuse 5 N d'hydroxyde de sodium, puis 19,5 ml d'eau. Les produits minéraux sont séparés par filtration et lavés par 2 fois 30 ml de chlorure de méthylène. Les filtrats sont rassemblés, séchés sur sulfate de magnésium, puis évaporés à sec. Le résidu obtenu, qui est constitué par le produit recherché, est acidifié par la quantité équivalente d'une solution d'acide chlorhydrique dans l'éthanol. On obtient 11,1 g de [(méthyl-1 pipéridyl-4)-2 éthyl]-4 phényl-2 quinoléine sous forme de monochlorhydrate fondant à 206°C.

Spectre R.M.N. du produit obtenu:

| | |
|---|---|
| aromatiques | δ: 7,3—8,6 ppm |
| $CH_2N$ et $CH_2Ar$ | δ: 2,6—3,6 ppm |
| $CH_3N$ | δ: 2,7 ppm |

L'[(éthoxycarbonyl-1 pipéridyl-4)-2 éthyl]-4 phényl-2 quinoléine est préparée comme suit:

Une solution de 15,6 g de phényl-2 [(pipéridyl-4)-2 éthyl]-4 quinoléine, préparée comme indiqué à l'exemple 7, dans 200 ml de chloroforme est traitée sous agitation par 200 ml d'une solution aqueuse 1 N d'hydroxyde de sodium. On ajoute ensuite, goutte-à-goutte, 21,7 g de chloroformiate d'éthyle et la suspension obtenue est agitée pendant 17 heures à température ambiante. Après séparation, la phase organique est lavée à l'eau, séchée sur sulfate de magnésium puis évaporée sous pression réduite. On obtient 20 g d'[(éthoxycarbonyl-1 pipéridyl-4)-2 éthyl]-4 phényl-2 quinoléine sous forme d'une huile.

Spectre R.M.N. du produit obtenu:

aromatiques $\delta$: 6,8—8,2 ppm

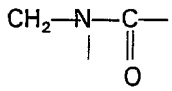 $\delta$: 3,8—4,2 ppm

Exemple 14

[(Chloro-2 phenyl)-2 quinolyl-4]-1 (piperidyl-4)-2 ethanone

On opère comme à l'exemple 2, en partant de 27 g d'hydrure de sodium (suspension à 50% dans l'huile de vaseline), de 36,7 g de (chloro-2 phényl)-2 quinoléinecarboxylate-4 de méthyle et de 32,1 g de (benzoyl-1 pipéridyl-4) acétate de méthyle. On obtient finalement 39,2 g de [(chloro-2 phényl)-2 quinolyl-4]-1 (pipéridyl-4)-2 éthanone, dont le chlorhydrate fond à 246°C.

Spectre R.M.N. du produit obtenu:

aromatiques $\delta$: 7,1—8,4 ppm

$CH_2N$ et $CH_2CO$ $\delta$: 2,3—3,1 ppm

Exemple 15

(Chloro-2 phenyl)-2 [(piperidyl-4)-2 ethyl]-4 quinoleine

On opère comme à l'exemple 9, sauf que l'on chauffe quatre heures à 160°C au lieu de 20 heures et que l'on part de la quantité de [(chloro-2 phényl)-2 quinolyl-4]-1 (pipéridyl-4)-2 éthanone équivalent à 17,1 g du chlorhydrate correspondant, 8 g, d'hydrate d'hydrazine à 80% et 7,1 g d'hydroxyde de potassium en pastilles dans 65 ml de diéthylène glycol. On obtient 9 g de (chloro-2 phényl)-2 [(pipéridyl-4)-2 éthyl]-4 quinoléine, dont le fumarate fond à 210°C.

Spectre R.M.N. du produit obtenu:

aromatiques $\delta$: 7,1—8,2 ppm

$CH_2N$ et $CH_2Ar$ $\delta$: 2,2—3,2 ppm

Exemple 16

[(Chloro-3 phenyl)-2 quinolyl-4]-1 (piperidyl-4)-2 ethanone

On opère comme dans l'exemple 2, en partant de 19,8 g d'hydrure de sodium (suspension à 50% dans l'huile), 27 g de (chloro-3 phényl)-2 quinoléinecarboxylate-4 de méthyle et 23,7 g de (benzoyl-1 pipéridyl-4) acétate de méthyle. On obtient 13,1 g de [(chloro-3 phényl)-2 quinolyl-4]-1 (pipéridyl-4)-2 éthanone, sous forme de méthanesulfonate qui fond à 210°C.

Exemple 17

(Chloro-3 phenyl)-2 [(piperidyl-4)-2 ethyl]-4 quinoleine

On opère comme dans l'exemple 9, en partant de 11,9 g de [(chloro-3 phényl)-2 quinolyl-4]-1 (pipéridyl-4)-2 éthanone préparée comme indiqué à l'exemple 16, 4,96 g d'hydrate d'hydrazine à 80% et 5,42 g d'hydroxyde de potassium dans 50 ml de diéthylène glycol. On obtient 7,2 g de (chloro-3 phényl)-2 [(pipéridyl-4)-2 éthyl]-4 quinoléine sous forme de monochlorhydrate fondant à 169°C.

Exemple 18

[(Pyridyl-4)-2 quinolyl-4]-1 (piperidyl-4)-2 ethanone

A 200 ml de tétrahydrofuranne sec, sous atmosphère d'azote, on ajoute lentement 30 ml d'une suspension à 20% d'hydrure de potassium dans l'huile. On introduit ensuite une solution de 15 g de (pyridyl-4)-2 quinoléinecarboxylate-4 d'éthyle dans 20 ml de tétrahydrofuranne sec, puis on ajoute lentement en deux heures une solution de 13 g de (benzoyl-1 pipéridyl-4) acétate de méthyle dans 60 ml de tétrahydrofuranne sec. On agite deux heures à température ambiante. On ajoute ensuite 30 ml d'éthanol, puis 10 ml d'eau et on évapore sous pression réduite. Le résidu est repris par 200 ml d'une solution aqueuse 5 N d'acide chlorhydrique et on chauffe au reflux pendant 12 heures. On extrait la phase aqueous par 3 fois 300 ml d'éther, puis l'alcalinise avec 150 ml d'une solution aqueuse 10 N

d'hydroxyde de sodium, et extrait par 3 fois 500 ml d'acétate d'éthyle. On lave la phase organique à l'eau et la sèche sur sulfate de magnésium. Après élimination du solvant par distillation sous pression réduite on obtient 9 g de produit brut. Ce produit est mis en solution dans l'éthanol et transformé, par addition d'une solution 5,8 N d'acide chlorhydrique dans l'éther, en dichlorhydrate de [(pyridyl-4)-2 quinolyl-4]-1 (pipéridyl-4)-2 éthanone, qui fond au dessus de 250°C.

Exemple 19

(Chloro-6 phenyl-2 quinolyl-4)-1 (piperidyl-4)-2 ethanone

On opère comme à l'exemple 18, en partant de 12,2 g de chloro-6 phényl-2 quinoléinecarboxylate-4 d'éthyle, 8,25 g de (benzoyl-1 pipéridyl-4) acétate de méthyle et 18 ml d'une suspension d'hydrure de potassium à 20% dans l'huile. On obtient 8,1 g de base brute, que l'on transforme en chlorhydrate, au sein de l'acétone, par addition d'une solution 4,3 N d'acide chlorhydrique dans l'éther. On obtient 6,5 g de monochlorhydrate de (chloro-6 phényl-2 quinolyl-4)-1 (pipéridyl-4)-2 éthanone, qui fond au-dessus de 260°C.

Exemple 20

(Piperidyl-4)-2 [(pyridyl-2)-2 quinolyl-4]-1 ethanone

A 200 ml de tétrahydrofuranne sec, sous atmosphère d'azote, on ajoute lentement 29 ml d'une suspension à 20% d'hydrure de potassium dans l'huile. On introduit ensuite une solution de 16,6 g de (pyridyl-2)-2 quinoléinecarboxylate-4 d'éthyle dans 20 ml de tétrahydrofuranne sec, puis on ajoute lentement en quatre heures une solution de 13 g de (benzoyl-1 pipéridyl-4) acétate de méthyle dans 100 ml de tétrahydrofuranne sec. On agite une heure à température ambiante. On ajoute ensuite 50 ml d'éthanol, puis 250 ml d'eau et évapore sous pression réduite. Le résidu brun est repris par 200 ml d'une solution aqueuse 12 N d'acide chlorhydrique et on chauffe au reflux pendant 12 heures. La solution acide est diluée par 300 ml d'eau, puis lavée par 2 fois 500 ml d'éther. Après évaporation de la phase aqueuse sous pression réduite, on obtient 28,8 g d'un mélange de chlorhydrate de la (pipéridyl-4)-2 [(pyridyl-2)-2 quinolyl-4]-1 éthanone, de chlorhydrates des acides (pyridyl-2)-2 quinoléine-carboxylique-4 et pipéridyl-4 acétique, et de chlorure de potassium. Ce mélange sera utilisé tel quel à l'exemple 24

Exemple 21

(Piperidyl-4)-2 [(thienyl-2)-2 quinolyl-4]-1 ethanone

On opère comme à l'exemple 18, en partant de 21,3 g de (thiényl-2)-2 quinoléinecarboxylate-4 d'éthyle, 16,2 g de (benzoyl-1 pipéridyl-4) acétate de méthyle et 37 ml d'une suspension à 20% d'hydrure de potassium dans l'huile. On obtient 17,7 g de [(thiényl-2)-2 quinolyl-4]-1 (pipéridyl-4)-2 éthanone brute, sous forme d'une huile.

Exemple 22

[(Piperidyl-4)-2 ethyl]-4 (pyridyl-4)-2 quinoleine

On chauffe à 180°C, pendant 15 minutes, un mélange de 6 g de [(pyridyl-4)-2 quinolyl-4]-1 (pipéridyl-4)-2 éthanone, préparée comme indiqué à l'exemple 18, et de 6 ml d'hydrate d'hydrazine à 85% dans 30 ml de diéthylène glycol. On refroidit à 50°C, puis on ajoute 6 g d'hydroxyde de potassium en pastilles et on chauffe à 180°C pendant deux heures. Après refroidissement, on dilue le mélange réactionnel avec de l'eau, et extrait par 4 fois 400 ml d'acétate d'éthyle. La phase organique est lavée par deux fois 500 ml d'eau, séchée sur sulfate de magnésium, et évaporée sous pression réduite. Le résidu obtenu est fixé sur une colonne de 150 g de silice, et on élue avec un mélange chloroforme-diéthylamine 9/1. On isole ainsi 3,1 g de [(pipéridyl-4)-2 éthyl]-4 (pyridyl-4)-2 quinoléine. Ce composé est mis en solution dans l'acétone et transformé, par addition d'une solution 6 N d'acide chlorhydrique dans l'éther, en son monochlorhydrate. Ce dernier présente, après recristallisation dans l'éthanol, un point de fusion de 224°C.

Exemple 23

Chloro-6 phenyl-2 [(piperidyl-4)-2 ethyl]-4 quinoleine

On opère comme à l'exemple 22, en partant de 5,3 g de (chloro-6 phényl-2 quinolyl-4)-1 (pipéridyl-4)-2 éthanone, préparée selon l'exemple 19, 2,3 ml d'hydrate d'hydrazine à 85% et 3 g d'hydroxyde de potassium. On obtient 5,2 g de produit brut, qui sont fixés sur une colonne de 50 g de silice. Après élution avec un mélange chloroforme-diéthylamine 9/1, on isole 2,2 g de chloro-6 phényl-2 [(pipéridyl-4)-2 éthyl]-4 quinoléine. Ce composé est dissous dans l'acétone et transformé, par addition d'une solution d'acide chlorhydrique dans l'éther, en son monochlorhydrate qui fond à 256°C.

Exemple 24

[(Piperidyl-4)-2 ethyl]-4 (pyridyl-2)-2 quinoleine

On procède comme à l'exemple 22, en partant de 16 g du mélange contenant la (pipéridyl-4)-2 [(pyridyl-2)-2 quinolyl-4]-1 éthanone préparé selon l'exemple 20, 11,8 ml d'hydrate d'hydrazine à 85% et 13,8 g d'hydroxyde de potassium. On obtient 14 g de produit brut, qui sont absorbés sur une

colonne de 500 g de silice. Après élution avec un mélange toluène-diéthylamine-méthanol 8/1/1, on isole 8,2 g de [(pipéridyl-4)-2 éthyl]-4 (pyridyl-2)-2 quinoléine, que l'on transforme, au sein de l'isopropanol, en son monochlorhydrate par addition d'une solution 4,5 N d'acide chlorhydrique dans l'éther. Ce monochlorhydrate présente un point de fusion de 211°C.

Exemple 25

[(Piperidyl-4)-2 ethyl]-4 (thienyl-2)-2 quinoleine

On procède comme à l'exemple 22, en partant de 17,7 g de (pipéridyl-4)-2 [(thiényl-2)-2 quinolyl-4]-1 éthanone préparée selon l'exemple 21, 9,4 ml d'hydrate d'hydrazine à 85% et 9 g d'hydroxyde de potassium. Le temps de chauffage à 180°C est porté à 6 heures après introduction de l'hydroxyde de potassium. On isole 16,5 g de produit brut que l'on fixe sur une colonne contenant 800 g de silice. Après élution avec un mélange chloroforme-diéthylamine 9/1 on isole 9,6 g de [(pipéridyl-4)-2 éthyl]-4 (thiényl-2)-2 quinoléine, que l on transforme, au sein de l'acétone, en son monochlorhydrate qui fond à 259°C.

Exemple 26

(Fluoro-4 phenyl)-2 [(piperidyl-4)-2 ethyl]-4 quinoleine

A 8 g de [(fluoro-4 phényl)-2 quinolyl-4]-1 (pipéridyl-4)-2 éthanone dans 180 ml d'eau, on ajoute 32 g de poudre de zinc, puis on coule en 3 heures 70 ml d'une solution aqueuse 12 N d'acide chlorhydrique. On laisse 12 heures sous agitation, puis on ajoute 10 g de zinc. On coule à nouveau, en 2 heures, 20 ml d'une solution aqueuse 12 N d'acide chlorhydrique, puis on agite pendant 2 heures. Le zinc est alors éliminé par filtration et lavé par de l'eau tiède. Le filtrat est refroidi, alcalinisé par addition de carbonate de potassium. L'insoluble est filtré, lavé par 3 fois 200 ml d'acétate d'éthyle. La phase aqueuse basique est extraite par 3 fois 300 ml d'acétate d'éthyle. Les phases organiques sont rassemblées, séchées sur sulfate de magnésium et évaporées sous vide. Le résidu est fixé sur une colonne de 480 g de silice, et on élue avec un mélange chloroformecyclohexane-diéthylamine 50/40/10. On isole ainsi 1,5 g de (fluoro-4 phényl)-2 [(pipéridyl-4)-2 éthyl]-4 quinoléine. Ce composé est mis en solution dans l'acétone et est transformé, par addition d'une solution 6 N d'acide chlorhydrique dans l'éther, en son monochlorhydrate fondant à 161°C.

Proprietes pharmacologiques

I — Affinite pour les sites recepteurs cerebraux des benzodiazepines

Cette affinité est mesurée par l'aptitude des produits à déplacer le Diazépam tritié ($^3$H Diazépam) de son site de liaison et est exprimée par une valeur $K_i$, en micromoles ($\mu$M), qui est calculée par la formule:

$$K_i = \frac{IC_{50}}{1 + \frac{C}{K_D}}$$

dans laquelle C représente la concentration de $^3$H Diazépam, $K_D$ une constante d'affinité égale à 2,74 $\mu$M et $IC_{50}$ la concentration nécessaire pour obtenir une inhibition de 50% de la liaison du $^3$H Diazépam.

Les produits one été testés selon le protocole de MOHLER et coll. Life Science, 1977, *20*, 2101. On a obtenu les résultats suivants:

| Produits | $K_i$ (µM) | Produits | $K_i$ (µM) |
|---|---|---|---|
| Exemple 2 | 8 | | |
| Exemple 3 | 4,5 | | |
| Exemple 1 | 0,5 | | |
| Exemple 6 | 2 | | |
| Exemple 5 | 5 | Exemple 12 | 0,5 |
| Exemple 4 | 1,6 | Exemple 17 | 0,6 |
| Exemple 9 | 1,3 | Exemple 18 | 4,5 |
| Exemple 8 | 0,4 | Exemple 22 | 0,16 |
| Exemple 7 | 0,2 | | |
| Exemple 10 | 0,6 | Exemple 23 | 0,37 |
| | | Exemple 24 | 0,02 |
| Exemple 13 | 0,1 | Exemple 26 | 0,16 |

II — Activité antiarythmique

L'activité antiarythmique des composés de la présente invention a été démontrée à l'aide du test à l'aconitine chez le rat.

Le principe de la technique repose sur le temps d'induction des arythmies ventriculaires provoquées par l'aconitine en perfusion lente chez le rat. Une substance antiarythmique retarde l'apparition des arythmies et le délai est proportionnel à l'activité de la molécule.

On utilise des groupes de 5 rats mâles. Une anesthésie individuelle est réalisée (uréthane 10%:1 g/kg/ip) pour permettre une cathétérisation de la veine du pénis. L'électrocardiogramme est enregistré. Au temps T = 0 la substance étudiée est injectée sous forme d'une solution aqueuse, à raison de 2,5 ml de solution par kg en 30 secondes. Au temps T = 90 secondes, soit 1 minute après la fin de l'injection, l'aconitine est perfusée à raison de 20 µg/1 minute, jusqu'à l'apparition d'extra systoles supra-ventriculaires. Le temps de perfusion de l'aconitrine est noté.

On exprime les résultats en $DE_{50}$, dose en mg/kg qui, par rapport aux animaux témoins, augmente de 50% le temps de perfusion de l'aconitine.

Les résultats obtenus sont rassemblés dans le tableau suivant:

| Produits | $DE_{50}$ mg/kg (i.v.) |
|---|---|
| Exemple 7 | 1,3 |

Proprietes toxicologiques

Les toxicités aiguës des composés selon l'invention ont été déterminées chez la souris mâle $CD_1$ (Charles RIVER) par voie orale. Les $DL_{50}$ one été calculés, après 3 jours d'observation, par la méthode cumulative de J. J. REED et H. MUENCH, (Amer, J. Hyg. 1938, *27*, 493).

Les compsés se comportent comme des substances relativement peu toxiques chez la souris, puisque les $DL_{50}$ des composés se situent entre 200 et 1000 mg/kg.

Utilisation therapeutique

Les composés de l'invention et leurs sels pharmaceutiquement acceptables peuvent être utilisés en thérapeutique humaine, sous forme de comprimés, capsules, gélules, suppositoires, solutions ingérables ou injectables, etc... comme antiarythmiques, hypnotiques, anticonvulsivants et pour le traitement des états d'anxiété et de divers états psychonévrotiques.

La posologie dépend des effets recherchés et de la voie d'administration utilisée. Par exemple, par voie orale, elle peut être comprise entre 5 et 250 mg de substance active par jour, avec des doses unitaires allant de 1 à 50 mg.

**Revendications**

1. Composés de formule générale:

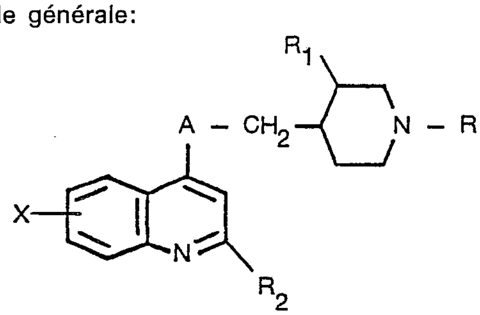

(I)

dans laquelle R est un atome d'hydrogène, un groupe alkyle 1 à 4 C, un groupe alcényle 3 ou 4 C, ou un groupe phénylalkyle dont la partie alkyle a un ou deux atomes de carbone, $R_1$ est un atome d'hydrogène, un groupe alkyle 1 à 4 C ou un groupe alcényle 2 à 4 C, $R_2$ est un groupe phényle, un groupe pyridyle, un groupe thiényle, ou un groupe phényle substitué par un ou deux substituants pris parmi les atomes d'halogène et les groupes alkyle ou alcoxy ayant 1 à 4 atomes de carbone, X représente un atome d'hydrogène ou un atome d'halogène fixé en position 6 sur le cycle de la quinoléine, et A représente un groupe CO ou $CH_2$, leurs diastéréoisomères, racémiques et énantiomères, et leurs sels d'addition avec les acides minéraux et organiques pharmaceutiquement acceptables.

2. Composés selon la revendication 1 dans lesquels $R_2$ est un groupe phényle ou phényle substitué et A représente $CH_2$.

3. Composé selon la revendication 2 de formule:

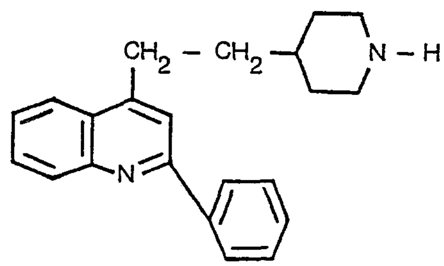

et ses sels d'addition avec les acides minéraux ou organiques pharmaceutiquement acceptables.

4. Procédé de préparation des composés selon la revendication 1 pour lesquels A représente le group $CH_2$, caractérisé en ce que l'on réduit les composés selon la revendication 1 correspondants pour lesquels A représente le groupe CO.

5. Procédé de préparation des composés selon la revendication 1 pour lesquels A représente le groupe CO et R l'atome d'hydrogène, caractérisé en ce que l'on condense un ester d'acide quinoléine-carboxylique-4 de formule:

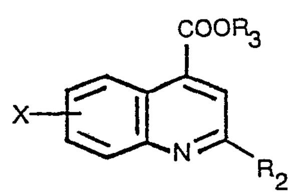

(II)

dans laquelle $R_3$ représente un groupe alkyle de bas poids moléculaire, avec un ester d'acide pipéridine-4 acétique de formule:

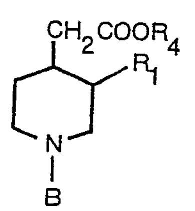

(III)

dans laquelle $R_4$ représente un groupe alkyle de bas poids moléculaire et B un groupe protecteur de la fonction amine, et hydrolyse le produit de condensation ainsi obtenu.

6. Procédé de préparation des composés selon la revendication 1 pour lesquels R est un groupe alkyle, alcényle ou phénylalkyle, caractérisé en ce que l'on fait réagir les composés selon la revendication 1 correspondants pour lesquels R est un atome d'hydrogène avec un agent alkylant.

7. Procédé pour la préparation des composés selon la revendication 1 pour lesquels A est un groupe $CH_2$ et R un groupe alkyle ou phénylalkyle, caractérisé en ce que l'on fait réagir les composés selon la revendication 1 correspondants pour lesquels A est un groupe $CH_2$ et R un atome d'hydrogène avec un composé de formule

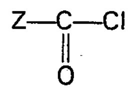

Z étant un groupe alkyle 1 à 3 C, un groupe phényle, un groupe phénylalkyle dont la partie alkyle a un atome de carbone ou un groupe alcoxy bas, et réduit ensuite au moyen d'un hydrure le composé ainsi obtenu.

8. Procédé de préparation des composés selon la revendication 1 pour lesquels A représente le groupe $CH_2$ et R l'atome d'hydrogène, caractérisé en ce que l'on fait réagir les composés de formule:

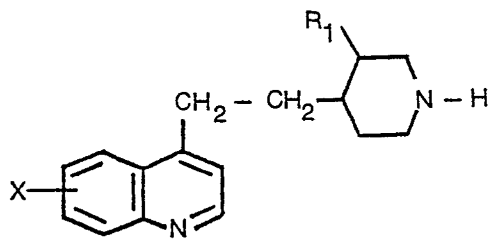

dans laquelle $R_1$ et X ont les mêmes significations que dans la formule (I) de la revendication 1, avec un composé permettant le remplacement de l'atome d'hydrogène fixé sur l'azote du cycle pipéridinique par un groupe B, stable en présence de composés organométalliques et susceptible d'être éliminé en milieu acide, en ce que l'on oxyde le composé de formule:

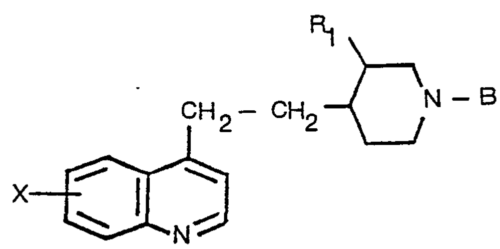

(VI)

ainsi obtenu, en ce que l'on fait réagir le composé de formule:

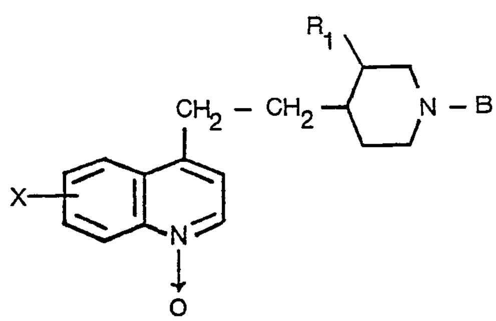

(VII)

ainsi obtenu avec un composé organométallique de formule $R_2M$ dans laquelle $R_2$ a les mêmes significations que dans la formule (I) de la revendication 1 et M représente un métal alcalin ou le radical MgHal, Hal désignant un atome d'halogène, et en ce que l'on hydrolyse le composé de formule:

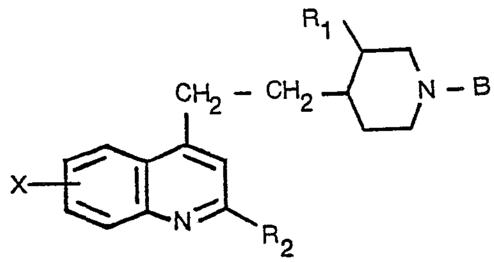

(VIII)

ainsi obtenu.

9. Procédé de préparation des composés selon la revendication 1 pour lesquels A est $CH_2$, R est l'atome d'hydrogène et $R_1$ est l'atome d'hydrogène ou un groupe alkyle 1 à 4 C, caractérisé en ce que l'on fait réagir le monochlorocarbène:CHCl sur un dérivé de l'indole de formule:

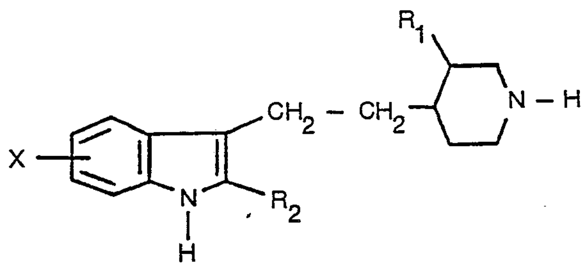

(IX)

dans laquelle $R_1$ est un atome d'hydrogène ou un groupe alkyle 1—4 C, et X et $R_2$ ont les mêmes significations que dans la formule (I) de la revendication 1.

10. Procédé de préparation des composés selon la revendication 1 pour lesquels R est l'atome d'hydrogène et $R_1$ le groupe vinyle et pour lesquels l'atome de carbone du cycle pipéridinique porteur du groupe $R_1$ a une configuration donnée, rectus ou sinister, caractérisé en ce que l'on chauffe, à température supérieure à 50°C, au sein d'un solvant protique ou d'un mélange de solvants protiques, en présence ou en l'absence de formaldéhyde, les composés selon la revendication 1 correspondants pour lesquels R est l'atome d'hydrogène, $R_1$ est le groupe vinyle et l'atome de carbone du cycle pipéridinique porteur du groupe $R_1$ a la configuration sinister ou rectus, partiellement ou totalement salifiés.

11. Procédé selon la revendication 10, caractérisé en ce que le chauffage est effectué à une température de 120 à 160°C dans un milieu aqueux acide, de pH voisin de 6.

12. Médicament, particulièrement utile comme antiarythmique, hypnotique, anticonvulsivant et pour le traitement des états psychonévrotiques et des états d'anxiété, caractérisé en ce qu'il contient, en tant que principe actif, un produit tel que défini dans chacune des revendications 1 et 2.

13. Médicament selon la revendication 12, caractérisé en ce qu'il contient, comme principe actif, le composé de formule:

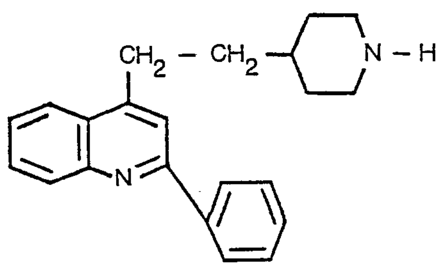

ou un sel de ce composé avec un acide pharmaceutiquement acceptable.

**Claims**

1. Compounds of the general formula:

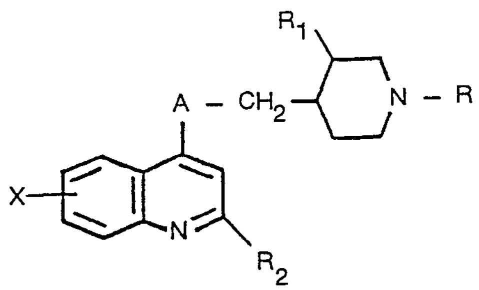

(I)

in which R is a hydrogen atom, an alkyl group having 1 to 4 carbon atoms, an alkenyl group having 3 or 4 carbon atoms or a phenylalkyl group in which the alkyl part has 1 or 2 carbon atoms, $R_1$ is a hydrogen atom, an alkyl group having 1 to 4 carbon atoms or an alkenyl group having 2 to 4 carbon atoms, $R_2$ is a phenyl group, a pyridyl group, a thienyl group, or a phenyl group which is substituted by one or two substituents from the group comprising halogen atoms and alkyl or alkoxy groups having 1 to 4 carbon atoms, X represents a hydrogen atom or a halogen atom bonded in position 6 on the quinoline ring and A represents a CO or $CH_2$ group, their diastereoisomers, racemates and enantiomers, and their additional salts with pharmaceutically acceptable mineral and organic acids.

2. Compounds according to Claim 1, in which $R_2$ is a phenyl group or a substituted phenyl group and A represents $CH_2$.

3. The compound according to Claim 2, of the formula:

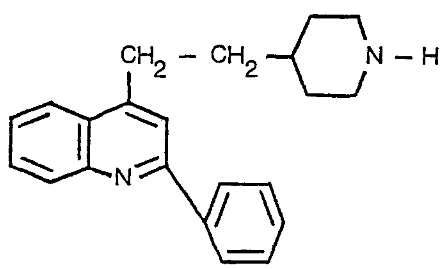

and its addition salts with pharmaceutically acceptable mineral or organic acids.

4. Process for the preparation of compounds according to Claim 1, in which A represents the $CH_2$ group, characterised in that the corresponding compounds according to Claim 1 in which A represents the CO group are reduced.

5. Process for the preparation of compounds according to Claim 1, in which A represents the CO group and R represents a hydrogen atom, characterised in that an ester of quinoline-4-carboxylic acid, of the formula:

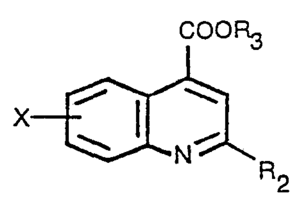

(II)

in which $R_3$ represents an alkyl group of low molecular weight, is subjected to a condensation reaction with a piperidine-4-acetic acid ester of the formula:

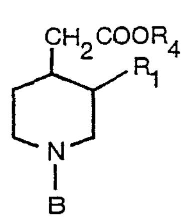

in which $R_4$ represents an alkyl group of low molecular weight and B represents a protective group on the amine function, and the condensation product thus obtained is hydrolysed.

6. Process for the preparation of compounds according to Claim 1, in which R is an alkyl, alkenyl or phenylalkyl group, characterised in that the corresponding compounds according to Claim 1 in which R is a hydrogen atom are reacted with an alkylating agent.

7. Process for the preparation of compounds according to Claim 1, in which A is a $CH_2$ group and R is an alkyl or phenylalkyl group, characterised in that the corresponding compounds according to Claim 1 in which A is a $CH_2$ group and R is a hydrogen atom are reacted with a compound of the formula

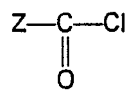

Z being an alkyl group having 1 to 3 carbon atoms, a phenyl group, a phenylalkyl group in which the alkyl part has 1 carbon atom, or a lower alkoxy group, and the compound thus obtained is then reduced with the aid of a hydride.

8. Process for the preparation of compounds according to Claim 1 in which A represents the $CH_2$ group and R represents a hydrogen atom, characterised in that the compounds of the formula:

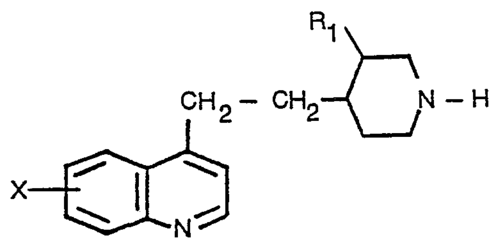

in which $R_1$ and X have the same meanings as in formula (I) of Claim 1, are reacted with a compound which permits replacement of the hydrogen atom bonded to the nitrogen of the piperidine ring by a group B, which is stable in the presence of organometallic compounds and capable of being removed in an acid medium, in that the compound of the formula:

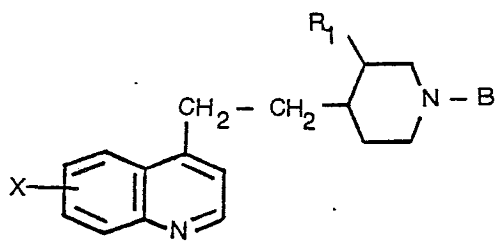

(VI)

thus obtained is oxidised, in that the compound of the formula:

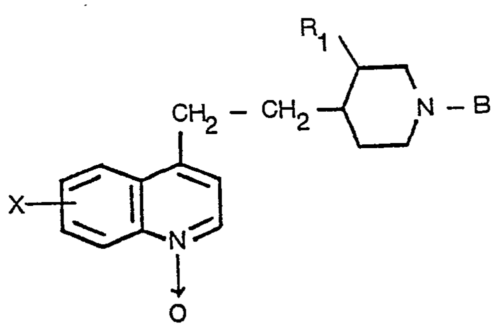

(VII)

thus obtained is reacted with an organometallic compound of the formula $R_2M$, in which $R_2$ has the same meanings as in formula (I) in Claim 1 and M represents an alkali metal or the MgHal radical, Hal designating a halogen atom, and in that the compound of the formula:

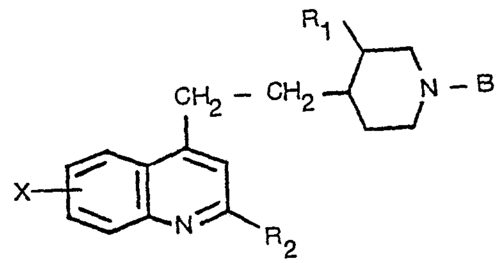

(VIII)

thus obtained in hydrolysed.

9. Process for the preparation of compounds according to Claim 1 in which A is $CH_2$, R is a hydrogen atom and $R_1$ is a hydrogen atom or an alkyl group having 1 to 4 carbon atoms, characterised in that monochlorocarbene:CHCl is reacted with an indole derivative of the formula:

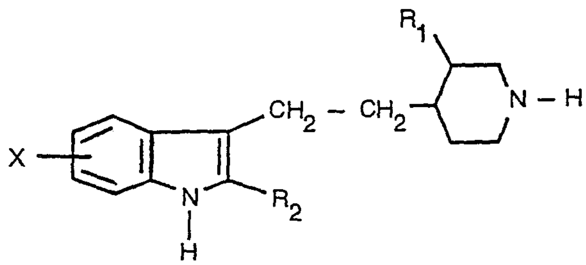

(IX)

in which $R_1$ is a hydrogen atom or an alkyl group having 1—4 carbon atoms and X and $R_2$ have the same meanings as in formula (I) of Claim 1.

10. Process for the preparation of compounds according to Claim 1 in which R is a hydrogen atom and $R_1$ is the vinyl group, and in which the piperidine ring carbon atom carrying the group $R_1$ has a given configuration, rectus or sinester, characterised in that the corresponding compounds according to Claim 1 in which R is a hydrogcn atom, $R_1$ is the vinyl group and the piperidine ring carbon atom carrying the group $R_1$ has the rectus or sinester configuration and which are partly or completely salified are heated at a temperature above 50°C in a protic solvent or a mixture of protic solvents, in the presence or absence of formaldehyde.

11. Process according to Claim 10, characterised in that the heating is carried out at a temperature 120 to 160°C in an aqueous acid medium with a pH of about 6.

12. Medicament, which can be used, in particular, as an antiarrhythmic, hypnotic or anti-convulsive agent and for the treatment of psychoneurotic states and anxiety states, characterised in that it contains, as the active principle, a product such as those defined in either of Claims 1 and 2.

13. Medicament according to Claim 12, characterised in that it contains, as the active principle, the compound of the formula:

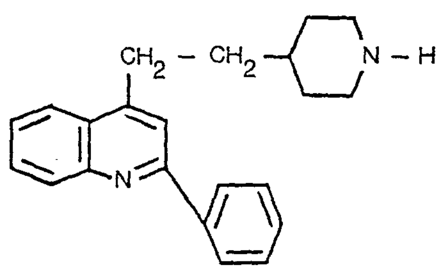

or a salt of this compound with a pharmaceutically acceptable acid.

**Patentansprüche**

1. Verbindungen der allgemeinen Formel (I)

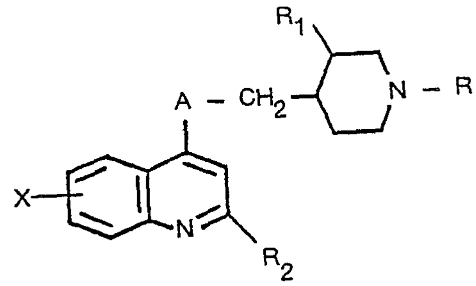

(I)

worin R ein Wasserstoffatom, eine Alkylgruppe mit 1 bis 4 Kohlenstoffatomen, eine Alkenylgruppe mit 3 oder 4 Kohlenstoffatomen oder eine Phenylalkylgruppe, deren Alkylteil ein oder 2 Kohlenstoffatome hat, ist, $R_1$ ein Wasserstoffatom, eine Alkylgruppe mit 1 bis 4 Kohlenstoffatomen oder eine Alkenylgruppe mit 2 bis 4 Kohlenstoffatomen ist, $R_2$ eine Phenylgruppe, eine Pyridylgruppe, eine Thienylgruppe oder eine Phenylgruppe, substituiert durch einen oder zwei Substituenten, genommen aus den Halogenatomen und den Alkyl- oder Alkoxygruppen mit 1 bis 4 Kohlenstoffatomen ist, X ein Wasserstoffatomen oder ein Halogenatom, fixiert in 6-Stellung des Chinolinrings bedeutet und A eine Gruppe CO oder $CH_2$ darstellt, ihre racemischen und enantiomeren Diastereoisomeren und ihre Säureadditionssalze mit pharmazeutisch annehmbaren Mineral- und organischen Säuren.

2. Verbindungen gemäß Anspruch 1, worin $R_2$ eine Phenyl- oder substituierte Phenylgruppe ist und A $CH_2$ bedeutet.

3. Verbindung gemäß Anspruch 2 der Formel

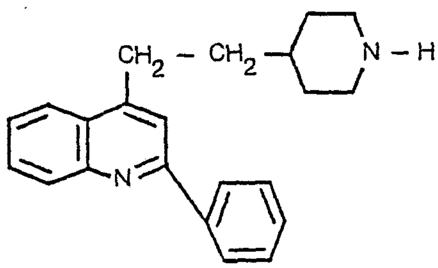

und ihre Säureadditionssalze mit pharmazeutisch annehmbaren Mineral- oder organischen Säuren.

4. Verfahren zur Herstellung der Verbindungen gemäß Anspruch 1, für die A die $CH_2$-Gruppe bedeutet, dadurch gekennzeichnet, daß man die entsprechenden Verbindungen gemäß Anspruch 1, für die A die CO-Gruppe bedeutet, reduziert.

5. Verfahren zur Herstellung der Verbindungen gemäß Anspruch 1, für die A die CO-Gruppe und R ein Wasserstoffatom bedeutet, dadurch gekennzeichnet, daß man einen Chinolin-4-carbonsäureester der Formel (II)

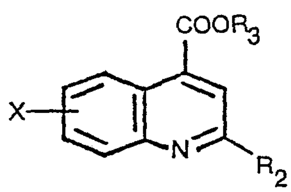

(II)

worin $R_3$ eine Alkylgruppe mit niedrigem Molekulargewicht bedeutet, mit einem Ester der 4-Piperidinessigsäure der Formel (III)

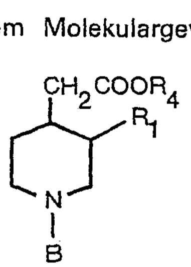

(III)

worin $R_4$ eine Alkylgruppe mit niedrigem Molekulargewicht und B eine Schutzgruppe der Aminfunktion bedeutet, kondensiert und das so erhaltene Kondensationsprodukt hydrolysiert.

6. Verfahren zur Herstellung der Verbindungen gemäß Anspruch 1, für die R eine Alkyl-, Alkenyl- oder Phenylalkylgruppe ist, dadurch gekennzeichnet, daß man die entsprechenden Verbindungen gemäß Anspruch 1, für die R ein Wasserstoffatom ist, mit einem Alkylierungsmittel zur Reaktion bringt.

7. Verfahren zur Herstellung der Verbindungen gemäß Anspruch 1, für die A eine $CH_2$-Gruppe und R eine Alkyl- oder Phenylalkylgruppe ist, dadurch gekennzeichnet, daß man die entsprechenden Verbindungen gemäß Anspruch 1, für die A eine $CH_2$-Gruppe und R ein Wasserstoffatom ist, mit einer Verbindung der Formel

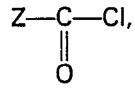

worin Z eine Alkylgruppe mit 1 bis 3 Kohlenstoffatomen, eine Phenylgruppe, eine Phenylalkylgruppe, deren Alkylteil ein Kohlenstoffatom oder eine niedriege Alkoxygruppe hat, zur Reaktion bringt und daß man dann die so erhaltene Verbindung mittels eines Hydrids reduziert.

8. Verfahren zur Herstellung der Verbindungen gemäß Anspruch 1, für die A eine $CH_2$-Gruppe und R ein Wasserstoffatom bedeutet, dadurch gekennzeichnet, daß man die Verbindungen der Formel

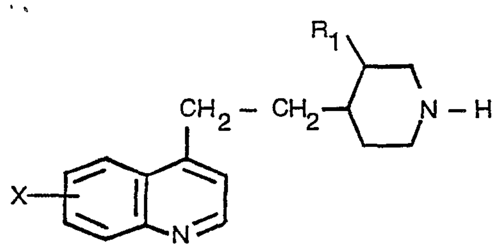

worin $R_1$ und X die gleichen Bedeutungen wie in Formel (I) von Anspruch 1 haben, mit einer Verbindung zur Reaktion bringt, die den Austausch des an das Stickstoffatom des Piperidinrings fixierten Wasserstoffatoms durch eine Gruppe B ermöglicht, die in Gegenwart von Organometallverbindungen stabil ist und in saurem Milieu entfernt werden kann, daß man die so erhaltene Verbindung der Formel (VI)

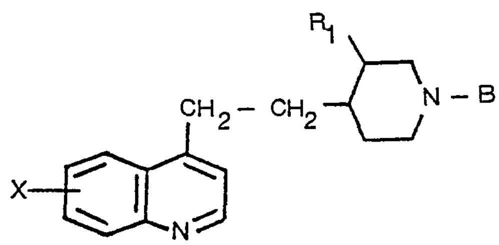

(VI)

oxidiert, daß man die so erhaltene Verbindung der Formel (VII)

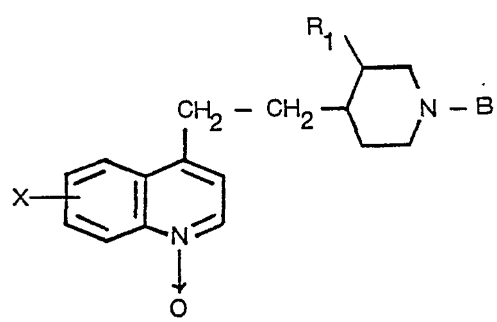

(VII)

mit einer Organometallverbindung der Formel $R_2M$ umsetzt, worin $R_2$ dieselben Bedeutungen wie in Formel (I) von Anspruch 1 hat und M ein Alkalimetall oder den Rest MgHal bedeutet, wobei Hal ein Halogenatom bedeutet, und daß man die so erhaltene Verbindung der Formel (VIII)

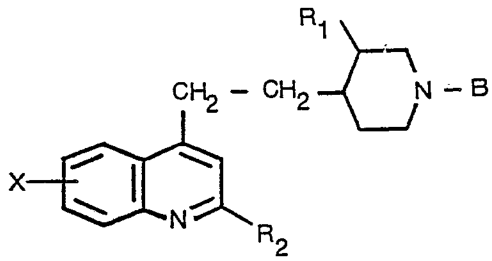

(VIII)

hydrolysiert.

9. Verfahren zur Herstellung der Verbindungen gemäß Anspruch 1, für die A $CH_2$, R ein Wasserstoffatom und $R_1$ ein Wasserstoffatom oder eine Alkylgruppe mit 1 bis 4 Kohlenstoffatomen ist, dadurch gekennzeichnet, daß man das Monochlorcarben:CHCl auf ein Indolderivat der Formel (IX)

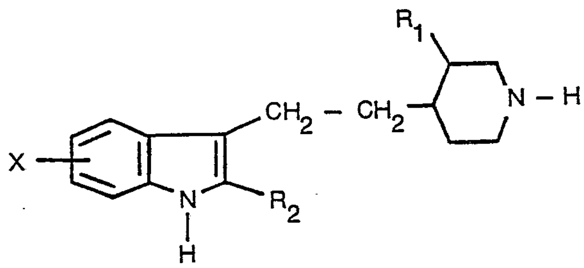

(IX)

einwirken läßt, worin $R_1$ ein Wasserstoffatom oder eine Alkylgruppe mit 1 bis 4 Kohlenstoffatomen ist und X und $R_2$ dieselben Bedeutungen haben wie in Formel (I) von Anspruch 1.

10. Verfahren zur Herstellung der Verbindungen gemäß Anspruch 1, für die R das Wasserstoffatom und $R_1$ die Vinylgruppe ist und für die das Kohlenstoffatom des Piperidinrings, das die Gruppe $R_1$ trägt, eine gegebene Konfiguration rectus oder sinester hat, dadurch gekennzeichnet, daß man die entsprechenden Verbindungen gemäß Anspruch 1, für die R das Wasserstoffatom ist, $R_1$ die Vinylgruppe ist und das Kohlenstoffatom des Piperidinrings, das die Gruppe $R_1$ trägt, die rectus oder sinesterkonfiguration hat, die teilweise oder ganz in Salzform sind, in einem protischen Lösungsmittel oder einem Gemisch protischer Lösungsmittel, in Gegenwart oder Abwesenheit von Formaldehyd, auf eine Temperatur oberhalb 50°C erhitzt.

11. Verfahren gemäß Anspruch 10, dadurch gekennzeichnet, daß das Erhitzen bei einer Temperatur von 120 bis 160°C, in einem sauren wäßrigen Milieu von einem pH nahe bei 6 bewirkt wird.

12. Medikament, insbesondere nützlich als Antiarrhythmikum, Hypnotikum, Antikonvulsivum und zur Behandlung von psychoneurotischen und Angstzuständen, dadurch gekennzeichnet, daß es als aktives Prinzip ein Produkt enthält, wie es in jedem der Ansprüche 1 und 2 definiert ist.

13. Medikament gemäß Anspruch 12, dadurch gekennzeichnet, daß es als aktives Prinzip die Verbindung der Formel

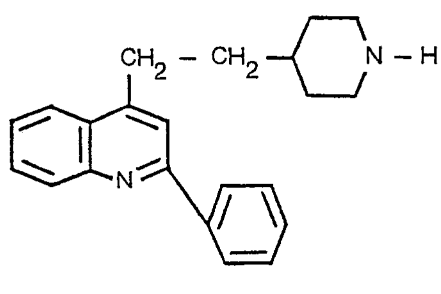

oder ein Salz dieser Verbindung mit einer pharmazeutisch annehmbaren Säure enthält.